# EUROPÄISCHE PATENTANMELDUNG

(11) **EP 0 636 604 A2**
(43) Veröffentlichungstag der Anmeldung: **01.02.1995**
(21) Anmeldenummer: 94111363.1
(22) Anmeldetag: 21.07.1994
(51) Int. Cl.: C07C 239/20, C08J 3/24, C08K 5/32, C08F 8/30

(54) **Verfahren zur Herstellung von weitgehend salzfreien wässrigen Lösungen von Bis-aminooxy-alkanen**

(30) Priorität: 29.07.1993 DE 4325455
(71) Anmelder: BASF Aktiengesellschaft, D-67063 Ludwigshafen (DE)
(72) Erfinder: Bauer, Gerhard, Dr., D-69469 Weinheim (DE); Baumstark, Roland, Dr., D-67434 Neustadt (DE); Bott, Kaspar, Dr., D-68165 Mannheim (DE); Voss, Hartwig, Dr., D-67227 Frankenthal (DE)

(57) **Zusammenfassung**

Herstellung von weitgehend salzfreien wäßrigen Lösungen von Bis-aminooxy-alkanen (I), indem man
a) ein Dihalogenalkan (II) mit einem Ketoxim (III) in der wäßrigen Lösung einer starken Mineralbase in den Alkylen-bis-oximether (IV) überführt,
b) den Alkylen-bis-oximether (IV) mit einer starken Mineralsäure und Wasser in das Keton und das mineralsaure Salz des Bis-aminooxy-alkans (I) spaltet und das Keton in an sich bekannter Weise abtrennt,
c) die verbleibende wäßrige Lösung zur Freisetzung des Bis-aminooxy-alkans (I) mit einem Alkalimetallhydroxid versetzt und
d) aus der so erhaltenen wäßrigen Lösung die ionischen Bestandteile durch Elektrodialyse entfernt.

## Beschreibung

Die vorliegende Erfindung betrifft ein neues Verfahren zur Herstellung weitgehend salzfreier Lösungen von Bis-aminooxy-alkanen (I).

Weiterhin betrifft die Erfindung salzarme Dispersionen aus vernetzbaren Polymeren und den freien Basen I als Vernetzungsmitteln sowie spezielle Teilschritte des Gesamtverfahrens.

Bis-aminooxy-alkane (I) sind allgemein bekannt und dienen als Vernetzungsmittel für Dispersionen von Additionspolymeren, Polykondensaten oder Polyaddukten, die reaktive Carbonylgruppen im Molekül enthalten (siehe DE-A 41 17 487 oder die ältere deutsche Anmeldung P 42 19 384.2).

Technisch die größte Bedeutung hat die Herstellung der Bis-aminooxy-alkane (I) durch Umsetzung N-geschützter Hydroxylamine mit Dihalogenalkanen (II) und anschließende Abspaltung der Schutzgruppen vom Hydroxylaminstickstoff.

Aus J. Chem. Soc., Seite 963 (1947) ist diese Umsetzung mit N-Hydroxyurethanen als N-geschützten Hydroxylaminen bekannt und in J. Org. Chem. 28, Seite 1604 (1963) ist die Verwendung von N-Hydroxyphthalimid für diesen Zweck beschrieben.

Diese Synthesemethoden vermögen jedoch aufgrund der niedrigen Ausbeuten und der Verluste an der Schutzgruppenverbindung nicht zu befriedigen.

Ferner fallen die Bis-aminooxy-alkane (I) in protonierter Form an, und es bereitet technische Schwierigkeiten, sie als wäßrige Lösungen der freien Basen I zu gewinnen, da bei dem hierzu erforderlichen Neutralisationsschritt ein wäßriges Gemisch aus den freien Basen I und Salzen entsteht.

Da jedoch der Salzgehalt der Bis-aminooxy-alkane (I) bei ihrer Verwendung als Vernetzer zur Koagulation der betreffenden Dispersionen führen kann, lag der Erfindung die Aufgabe zugrunde, ein Verfahren zur Herstellung von weitgehend salzfreien wäßrigen Lösungen von Bis-aminooxy-alkanen (I) sowie Dispersionen vernetzbarer Polymerer, welche diese Produkte enthalten, bereitzustellen.

Demgemäß wurde ein Verfahren zur Herstellung von weitgehend salzfreien wäßrigen Lösungen von Bis-aminooxy-alkanen (I) gefunden, welches dadurch gekennzeichnet ist, daß man
a) ein Dihalogenalkan (II) mit einem Ketoxim (III) in der wäßrigen Lösung einer starken Mineralbase in den Alkylen-bis-oximether (IV) überführt,
b) den Alkylen-bis-oximether (IV) mit einer starken Mineralsäure und Wasser in das Keton und das mineralsaure Salz des Bis-aminooxy-alkans (I) spaltet und das Keton in an sich bekannter Weise abtrennt,
c) die verbleibende wäßrige Lösung zur Freisetzung des Bis-aminooxy-alkans (I) mit einem Alkalimetallhydroxid versetzt und
d) aus der so erhaltenen wäßrigen Lösung die ionischen Bestandteile durch Elektrodialyse entfernt.

Außerdem wurden weitgehend salzfreie Polymerdispersionen gefunden, welche die Verbindung (I) enthalten. Daneben erstreckt sich die Erfindung auf besonders zweckmäßige Ausführungsformen einiger Stufen des Gesamtverfahrens.

Das erfindungsgemäße Verfahren läßt sich in einem einfachen Fall wie folgt veranschaulichen:
a) 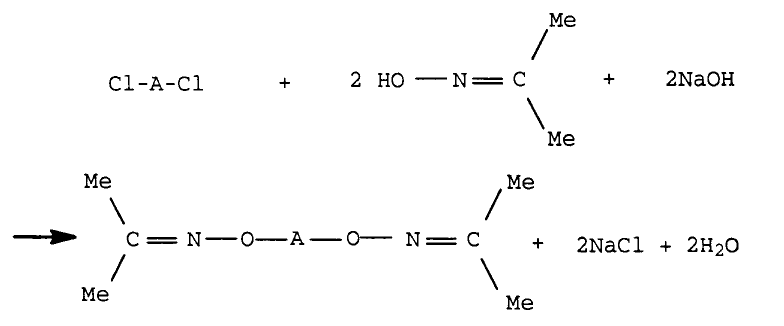
b) 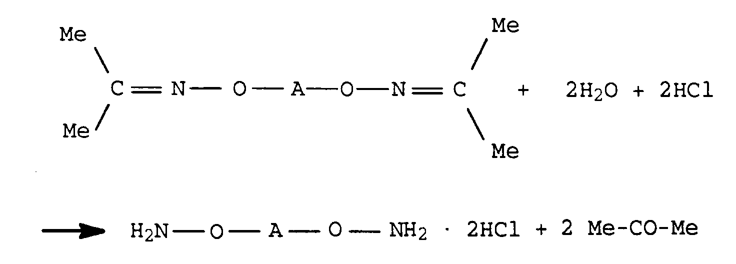
c)

   H₂N―O―A―O―NH₂ · 2 HCl + 2 NaOH → H₂N―O―A―O―NH₂ + 2 NaCl + 2 H₂O

   A = Alkylen
d) Elektrodialyse der wäßrigen Produktlösungen aus Stufe c) zur weitgehenden Entfernung von Natriumchlorid.

Die Stufen a) bis c) können in an sich bekannter Weise vorgenommen werden. Besonders zweckmäßige neue Ausführungsformen sind weiter unten beschrieben.

Unter den Verfahrensprodukten sind diejenigen bevorzugt, die sich von unverzweigten C₂- bis C₁₂-Alkanen, insbesondere von Butan ableiten. Als entsprechende Dihalogenalkane (II) kommen in erster Linie die α,ω-Dichlor- und daneben die α,ω-Dibromverbindungen in Betracht.

Unter den Ketoximen (III) sind solche der Formel 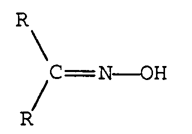
besonders geeignet, in der die Reste R gleich oder verschieden sind und für je einen C₁- bis C₁₀-organischen Rest, vorzugsweise für eine C₁- bis C₄-Alkylgruppe wie die n-Propyl-, iso-Propyl-, n-Butyl-, tert.-Butylgruppe oder vor allem die Methyl- oder die Ethylgruppe stehen, wobei als Ketoxim (III) Acetonoxim besonders bevorzugt ist.

Die Ketoxime (III) sind bekannt oder können nach bekannten Methoden, beispielsweise durch Umsetzung der entsprechenden Ketone mit Hydroxylamin, erhalten werden.

Das Molverhältnis von Ketoxim (III) zu Dihalogenalkan (II) beträgt vorzugsweise 2:1 bis 3:1, insbesondere 2:1 bis 2,2:1.

Als wäßrige Lösungen einer starken Mineralbase eignen sich wäßrige Alkali- oder Erdalkalimetallhydroxid-Lösungen, wobei aus wirtschaftlichen Gründen Natriumhydroxid und Calciumhydroxid bevorzugt sind. Die schwerlöslichen Erdalkalimetallhydroxide kann man auch in Form ihrer Suspension einsetzen. Pro Mol Ketoxim (III) setzt man zweckmäßigerweise 1 bis 2, insbesondere 1 bis 1,5 Äquivalente des Hydroxids ein.

Im allgemeinen ist es zur Erzielung befriedigender Ausbeuten erforderlich, die Umsetzung der Dihalogenalkane (II) mit den Ketoximen (III) in einem Lösungsmittel vorzunehmen, wobei wasserlösliche Lösungsmittel, beispielsweise Dimethylformamid oder Dimethylsulfoxid bevorzugt sind, da sie eine hinreichende Löslichkeit der polaren Reaktionsteilnehmer in der organischen Phase gewährleisten.

Es wurde jedoch gefunden, daß man die Mitverwendung eines Phasentransferkatalysators den Einsatz eines Lösungsmittels ganz oder weitgehend entbehrlich macht.

Als Phasentransferkatalysatoren können quartäre Ammonium- oder Phosphoniumsalze, vorzugsweise Tetraalkyl- oder Trialkylbenzylammonium- bzw. -phosphoniumsalze eingesetzt werden. Besonders bevorzugt sind die Triethyl-, Tributylbenzyl- und Tetrabutylammoniumchloride, -bromide und -hydrogensulfate sowie Tributylhexadecylphosphoniumbromid.

Den Phasentransferkatalysator setzt man in der Regel in Mengen von 0,5 bis 2, vorzugsweise 0,7 bis 1 mol-%, bezogen auf das Ketoxim (III) ein.

Im Falle des Acetonoxims wurde gefunden, daß die Umsetzung allein mit einem Phasentransferkatalysator und ohne Mitverwendung nennenswerter Mengen eines Lösungsmittels besonders gut gelingt. Diese Ausführungsform ist für sich allein und im Rahmen des Gesamtverfahrens besonders bevorzugt, weil bei ihr der technische Aufwand, der durch das Lösungsmittel bedingt wird, entfällt.

Die Umsetzung der Dihalogenalkane (II) mit den Ketoximen (III) führt man üblicherweise bei Temperaturen von 40 bis 120, meistens von 60 bis 105°C, und Drücken von 0,5 bis 2, vorzugsweise bei Normaldruck durch.

Die Reaktionszeiten betragen normalerweise 1 bis 10, meistens jedoch 4 bis 5 Stunden.

Verfahrenstechnisch geht man in der Regel so vor, daß man das Dihalogenalkan (II), die wäßrige starke Mineralbase und den Phasentransferkatalysator und gegebenenfalls das Lösungsmittel auf die Reaktionstemperatur erhitzt und dann das Ketoxim (III) zudosiert.

Bei der Umsetzung erhält man eine salzhaltige wäßrige Phase und eine organische Phase, die aus dem Alkylen-bis-oximether (IV) besteht oder diesen enthält. Die organische Phase, gegebenenfalls nach Abtrennung des organischen Lösungsmittels, kann in dieser Form in der nächsten Verfahrensstufe eingesetzt werden, in welcher man den Alkylen-bis-oximether (IV) mit einer wäßrigen starken Mineralsäure in das mineralsaure Salz des Bis-aminooxy-alkans (I) und das Keton spaltet.

Für diese Reaktion geeignete Mineralsäuren sind Schwefelsäure, Salpetersäure, Phosphorsäure und besonders Salzsäure, und zwar vorzugsweise in wäßriger Lösung.

Diese starken Mineralsäuren werden in der Regel im Molverhältnis von 2:1 bis 6:1, zweckmäßigerweise von 2:1 bis 4:1, bezogen auf den Alkylen-bis-oximether (IV), verwendet.

Die Spaltung des Alkylen-bis-oximethers (IV) führt man im allgemeinen bei Temperaturen von 80 bis 110 °C und Drücken von 0,5 bis 1,5, vorzugsweise von 0,7 bis 1 bar durch. Die Reaktionszeiten betragen üblicherweise 2 bis 10, insbesondere 3 bis 5 Stunden.

In einer bevorzugten Ausführungsform des erfindungsgemäßen Verfahrens, die auch für sich allein besonders vorteilhaft ist, geht man von Alkylen-bis-acetonoximethern aus und hydrolysiert diese mit wäßriger Salzsäure. Das dabei entstehende Aceton entfernt man laufend aus dem Reaktionsgemisch, wobei Chlorwasserstoff und Wasser teilweise mit übergehen können.

Die Aufarbeitung erfolgt in diesem Fall in der Weise, daß man Chlorwasserstoff und Wasser, die im Reaktionsgefäß verblieben sind, zweckmäßigerweise unter Zuhilfenahme eines Schleppmittels, vollständig entfernt, das verbleibende mineralsaure Salz des Bis-aminooxy-alkans (I) in Wasser aufnimmt und das Schleppmittel abtrennt.

Als Schleppmittel eignen sich Benzol, Tetrachlorkohlenstoff und vor allem Cyclohexan.

Die verbleibende wäßrige Salzlösung wird zur Freisetzung des Bis-aminooxy-alkans (I) mit einem Alkalimetallhydroxid versetzt, wobei als Alkalimetallhydroxid Kaliumhydroxid und Natriumhydroxid besonders bevorzugt sind.

In Hinblick auf die erfindungsgemäß in der nachfolgenden Verfahrensstufe durchzuführende Elektrodialyse nimmt man die Freisetzung des Bis-aminooxy-alkans (I) normalerweise mit stöchiometrischen oder leicht überstöchiometrischen Mengen Alkalimetallhydroxid, bezogen auf die Menge des I-Salzes, vor, um so eine unnötige Erhöhung des Elektrolytgehalts der Lösung zu vermeiden.

Die Neutralisation wird in an sich bekannter Weise durchgeführt, so daß sich nähere Erläuterungen hierzu erübrigen.

Nach der Neutralisation mit dem Alkalimetallhydroxid wird die wäßrige Lösung mit dem Bis-aminooxy-alkan (I) und dem Alkalimetallsalz zur Abtrennung der ionischen Bestandteile erfindungsgemäß der Elektrodialyse unterworfen.

Die Entsalzung wäßriger Salzlösungen mittels Elektrodialyse und entsprechende Elektrodialyseapparaturen sind im Prinzip bekannt und z.B. in H. Strathmann "Trennung von molekularen Mischungen mit Hilfe synthetischer Membranen", Steinkopf Verlag, Darmstadt, 1979, Seite 76 bis 86, und in D.S. Flett "Ion Exchange Membranes", Ellis Horwood, Chichester 1983, Seite 179 bis 191, beschrieben.

Der Entsalzungsschritt des erfindungsgemäßen Verfahrens wird zweckmäßigerweise so durchgeführt, daß man zwischen zwei Elektroden alternierend Anionen- und Kationenaustauschermembranen parallel zueinander anordnet und die durch zwischengelegte Distanzrahmen gebildeten Kammern gegeneinander abdichtet. Ein Element, bestehend aus einer Diluatkammer, einer Konzentratkammer, einer Anionen- und einer Kationenaustauschermembran, wird als Teilzelle bezeichnet.

Die salzhaltige Produktlösung (im folgenden auch als "Diluat" bezeichnet), wird durch diejenigen Kammern geführt, die in Richtung der Anode durch eine Anionenaustauschermembran begrenzt werden. Eine wäßrige elektrolythaltige Lösung (im folgenden auch als "Konzentrat" bezeichnet), deren Anfangsleitfähigkeit z.B. zwischen 1 mS/cm und 10 mS/cm liegt, wird zur Aufnahme des abzutrennenden Salzes durch diejenigen Kammern geführt, die in Richtung der Anode durch eine Kationenaustauschermembran begrenzt werden. Als Elektrolyt wird im Konzentrat in der Regel das Salz verwendet, welches aus dem Diluat abgetrennt werden soll.

Von den Diluat- und Konzentratkammern durch die jeweils letzte Membran, vorzugsweise eine Kationenaustauschermembran, getrennt, befindet sich der Kathoden- bzw. Anodenraum. An den Elektroden wird vorteilhafterweise während des Elektrodialyseprozesses eine elektrolythaltige Lösung vorbeigeführt, um entstehende Gase aus den Elektrodenkammern auszutragen. Als Elektrodenspülung verwendet man zweckmäßigerweise eine 1- bis 10 gew.-%ige wäßrige Natriumsulfat-Lösung.

Als Ionenaustauschermembranen verwendet man handelsübliche, für Anionen und Kationen selektive, Membranen. Sie weisen im allgemeinen eine Permselektivität von über 0,9 und einen elektrischen Widerstand von unter 5 Ωcm² auf (vgl. Desalination, Band 34, S. 77 bis 95 (1980)). Die Ionenaustauschermembranen bestehen im allgemeinen aus einer Trägerfolie oder einem Trägergewebe aus einem Polyester, Polyethylen oder Polyvinylchlorid, auf welche die fertigen Austauscherharze aufgebracht, oder auf denen das Austauscherharz erst wie üblich polymerisiert und anschließend weiterbehandelt wurde, um Kationen- oder Anionenaustauscher zu erhalten (vgl. hierzu Ullmanns Encyklopädie der technischen Chemie, 4. Aufl., Band 13, S. 279 ff. (1977). Insbesondere sind stark saure Kationenaustauscher oder stark basische Anionenaustauscher auf Basis von vernetzten, durch Sulfonsäuregruppen oder quartäre Ammoniumgruppen modifizierten Styrol-Butadien- oder Styrol-Divinylbenzol-Copolymeren auf einem Polyvinylchlorid-Stützgewebe zu nennen.

Man elektrodialysiert beim erfindungsgemäßen Verfahren im allgemeinen bei einer Temperatur von unter 100°C, vorzugsweise bei 15 bis 80°C, und bei einer Stromdichte, die in der Regel 3000 A/m² nicht übersteigt, vorzugsweise bei 10 bis 1000 A/m². Die für den Ionentransport durch die Membranen erforderliche Gleichspannung richtet sich dabei nach Art und Konzentration der Ionen. Bedingt durch den elektrischen Widerstand der Elektrodialysezelle ergeben sich bei den obengenannten Stromdichten Spannungen von bis zu 3 V pro Teilzelle. Die Membranen haben dabei üblicherweise einen Abstand von 2,0 bis 0,4 mm.

Das erfindungsgemäße Verfahren kann sowohl kontinuierlich in mehreren hintereinander geschalteten Membranstapeln als auch diskontinuierlich durch Kreislaufführung der Flüssigkeitsströme unter Zuhilfenahme von Puffergefäßen oder durch Mischformen dieser Verfahrensarten ausgeführt werden.

Damit die Elektrodialyse ohne Probleme durchgeführt werden kann, empfiehlt es sich, die Produktlösung auf einen pH-Wert zu bringen, der dem pH-Wert des freien Bis-aminooxy-alkans (I) in wäßriger Lösung entspricht oder bis zu etwa einer Einheit höher liegt.

Weiterhin sollten Oligomere, die bei der Spaltung des Alkylen-bis-oximethers (IV) entstehen, abgetrennt werden. Dies kann durch Filtration erfolgen, nachdem die wäßrige Lösung des mineralsauren Salzes des Bis-aminooxy-alkans (I) neutralisiert wurde, da die Nebenprodukte dabei zum großen Teil ausfallen. Zur Verbesserung der Abtrennung kann vor oder nach der Fällung Aktivkohle zugesetzt werden.

Der Grad der Abreicherung der ionischen Bestandteile läßt sich in weiten Grenzen einstellen. Der hierzu erforderliche Aufwand, vor allem Dauer und Strombedarf der Elektrodialyse, hängt im wesentlichen von der anfänglichen Salzkonzentration im Diluat und der Stromausbeute ab.

Sollen die wäßrigen Lösungen der Bis-aminooxy-alkane (I) als Vernetzungsmittel enthaltender Zusatz zu Dispersionen dienen, so senkt man den Gehalt an mineralsauren Salzen von Alkalimetallen in dieser Lösung elektrodialytisch unter 20 mol.-%, vorzugsweise unter 10 mol-%, bezogen auf das Bis-aminooxy-alkan (I).

Die elektrodialytische Abreicherung der ionischen Bestandteile aus wäßrigen Lösungen der Bis-aminooxy-alkane (I) ist im übrigen nicht an die Provenienz der Lösungen gebunden, d.h. sie eignet sich zur Entfernung von Salzen aus beliebigen wäßrigen Lösungen von Bis-aminooxy-alkanen (I) und Alkalimetallsalzen.

Die erfindungsgemäßen, weitgehend salzfreien wäßrigen Lösungen der Bis-aminooxy-alkane (I) können unmittelbar als Zusatz zu wäßrigen Dispersionen von vernetzbaren Polymeren verwendet werden, die im Molekül Carbonylgruppen in Aldehyd- oder Ketonfunktion enthalten. Da sie praktisch keine Salze mehr enthalten, kann man sie den Polymerdispersionen ohne weiteres zusetzen, ohne auch bei elektrolytlabilen Systemen deren kolloidale Stabilität hierdurch zu beeinträchtigen.

Aufgrund des geringen Salzgehaltes erhält man aus den Dispersionen klare Filme.

Als Polymere eignen sich Polykondensate oder Polyaddukte, welche Carbonylgruppen in Aldehyd- oder Ketonfunktion enthalten. Bevorzugt sind jedoch Additionspolymere aus olefinisch ungesättigten Verbindungen, welche mit dem reaktiven Carbonylgruppen funktionalisiert sind.

Man erhält diese Additionspolymeren durch Copolymerisation von Monomeren, die eine Carbonylgruppe in Aldehyd- oder Ketonfunktion enthalten (Monomere A), mit sonstigen Monomeren (Monomere B).

Geeignete Monomere A sind Acrolein, Methacrolein, Vinylalkylketone, Formylstyrole, ferner Acrylsäure- oder Methacrylsäurealkylester bzw. N-alkylsubstituierte -amide, die im Alkylteil eine Carbonylgruppe in Aldehyd- oder Ketonfunktion enthalten.

Besonders bevorzugt sind 2-(3-Oxo-butyryloxy)-ethylmethacrylat, ("Acetoacetoxyethylmethacrylat") und insbesondere N-(1,1-Dimethyl-3-oxo-butyl)-acrylamid ("Diacetonacrylamid").

Geeignete Monomere B sind die Ester der Acryl- oder Methacrylsäure, vorzugsweise die Alkylester, wobei die C₁- bis C₁₀-Alkylester wie Methylacrylat, Ethylacrylat, n-Butylacrylat, 2-Ethyl-hexylacrylat und Methylmethacrylat sowie Mischungen dieser Monomeren besonders bevorzugt sind.

Ferner kommen als Monomere B Carbonsäurevinylester, vorzugsweise von C₁- bis C₂₀-Carbonsäuren wie Vinylpropionat oder Vinyllaurat in Betracht, daneben Vinylaromaten mit bis zu 20 C-Atomen, vorzugsweise Styrol, außerdem olefinisch ungesättigte Nitrile, Vinylhalogenide oder aliphatische C₄- bis C₈-Kohlenwasserstoffe mit mindestens zwei konjugierten olefinischen Doppelbindungen, insbesondere Butadien, Isopren und Chloropren.

Weiterhin eignen sich als Monomere B Methacrylamide, hydroxyfunktionelle Monomere oder Monomere mit salzbildenden Gruppen, insbesondere mit Carboxylgruppen wie Acrylsäure und Methacrylsäure.

Die Polymere enthalten normalerweise 0,001 bis 20, insbesondere 0,01 bis 10 und vor allem 0,05 bis 5 Gew.-% Carbonylgruppen in Keton- oder Aldehydfunktion. Sie können durch Substanz- oder Suspensionspolymerisation, vorzugsweise jedoch durch Emulsions- oder Lösungspolymerisation hergestellt werden.

Die Emulsionspolymerisation dient vor allem zur Herstellung wäßriger Primärdispersionen. Man bringt dabei in der Regel die Monomeren mit einem wasserlöslichen Initiator in Gegenwart eines Emulgators bei Temperaturen von 30 bis 90°C in Wasser zur Reaktion.

Geeignete Initiatoren sind z.B. Natrium-, Kalium- und Ammoniumpersulfat, tert.-Butylhydroperoxid sowie wasserlösliche Azoverbindungen oder auch Redoxinitiatoren.

Als Emulgatoren können z.B. Alkalisalze von längerkettigen Fettsäuren, des weiteren Alkylsulfate, Alkylsulfonate, alkylierte Arylsulfonate oder alkylierte Biphenylethersulfonate dienen.

Des weiteren kommen als Emulgatoren Umsetzungsprodukte von Alkylenoxiden, insbesondere Ethylen- oder Propylenoxid mit Fettalkoholen, Fettsäuren, Phenol oder Alkylphenolen in Betracht.

Für wäßrige Sekundärdispersionen wird das Polymere zunächst durch Lösungspolymerisation bzw. Lösungspolykondensation in einem organischen Lösungsmittel hergestellt und anschließend in Wasser dispergiert.

Enthalten die Polymeren stark hydrophile Gruppen wie die Carboxylgruppe, sind sie in aller Regel selbstdispergierend, so daß sich die für manche Anwendungszwecke nachteilige Mitverwendung sonstiger Emulgatoren erübrigt.

Der Dispersion wird zum Zweck der Vernetzung ein Bis-aminooxy-alkan (I) in Form einer weitgehend salzfreien wäßrigen Lösung zugesetzt.

Das Vernetzungsmittel kann der Dispersion im Unter- oder Überschuß, vorzugsweise jedoch in stöchiometrischer Menge, bezogen auf die zu vernetzenden Carbonylgruppen in Keton- oder Aldehydfunktion, zugesetzt werden.

Die Dispersionen können noch weitere Komponenten wie Filmbildungshilfsmittel, Füllstoffe oder Fungizide enthalten.

Der Feststoffgehalt der erfindungsgemäßen Dispersionen liegt bevorzugt zwischen 20 und 90, insbesondere zwischen 30 und 70 Gew.-%.

Im übrigen ist dem Fachmann die Herstellung solcher Dispersionen bekannt (vgl. die ältere deutsche Anmeldung P 42 19 384.2), so daß weitergehende Erläuterungen hierzu nicht erforderlich sind.

Die erfindungsgemäßen Dispersionen eignen sich beispielsweise als Dicht-, Kleb- und Anstrichstoffe.

### Beispiel 1

### Herstellung von Butylen-1,4-bis-acetonoximether (Stufe a)

Eine Mischung aus 576 g (4,53 mol) 1,4-Dichlorbutan, 1080 g 50%iger (13,5 mol) Natronlauge, 270 g Wasser und 36 g 50%iger (65 mmol) Tetrabutylammoniumchlorid-Lösung in Wasser wurde auf 50°C erhitzt und innerhalb einer Stunde im Zulaufverfahren mit 657 g (9,00 mol) Acetonoxim versetzt. Nach dreistündigem Erhitzen unter Rückfluß wurde das ausgefallene Natriumchlorid mit 1500 g Wasser gelöst, und danach wurden die Phasen getrennt. Nach Destillation der organischen Phase erhielt man den Oximether in einer Ausbeute von 65 %.

### Beispiel 2

### Herstellung des Hydrochlorids von 1,4-Bis-aminooxy-butan (Stufe b)

500 g (2,5 mol) Butylen-1,4-bis-acetonoximether und 1000 g Wasser wurden zum Sieden erhitzt und innerhalb einer Stunde im Zulaufverfahren mit 1000 g konzentrierter (10 mol) Salzsäure versetzt. Während der Zulaufzeit sowie dem anschließenden vierstündigen Sieden wurden das entstehende Aceton und untergeordnet Chlorwasserstoff und Wasser laufend abdestilliert.

Bei der anschließenden Aufarbeitung wurden die Restmengen Chlorwasserstoff und Wasser destillativ mittels Cyclohexan als Schleppmittel entfernt, das Hydrochlorid des 1,4-Bis-aminooxy-butans in Wasser aufgenommen und Cyclohexan abgetrennt. Man erhielt das Hydrochlorid in einer Ausbeute von 95,3 %.

### Beispiel 3

### Herstellung einer weitgehend salzfreien Lösung von 1,4-Bis-aminooxy-butan (Stufen c und d)

Die Entsalzungen erfolgten diskontinuierlich.

### a) Apparatur

Die Apparatur bestand aus einer Elektrodialyse-Zelle (ED-Zelle) mit drei Kreisläufen (Diluat-, Konzentrat- und Elektrodenspülkreislauf). Jeder dieser Kreisläufe war mit einer Magnetkreiselpumpe, einem Wärmetauscher und einem Vorratsbehälter ausgerüstet und über Schläuche mit der ED-Zelle verbunden.

Die ED-Zelle hatte zwei Platinelektroden, die jeweils eine Fläche von 35 cm² aufwiesen. Die Elektrodenräume waren von den angrenzenden Konzentratkammern durch Kationenaustauschermembranen vom Typ Nafion® (Fa. DuPont) abgetrennt. Außerdem waren die Elektrodenkammern mit dem Elektrodenspülkreis verbunden. Zwischen den Elektrodenkammern befanden sich 11 Konzentrat- und 10 Diluatkammern in alternierender Anordnung. Die Kammern waren voneinander abwechselnd durch Kationenaustauschmembranen vom Typ Selemion® CMV bzw. Anionenaustauschermembranen vom Typ Selemion® AMV (Fa. Asahi Glass) getrennt. Alle Membranen hatten eine aktive Fläche von 37 cm². Die Membranabstände betrugen 1 mm. Zulauf und Ablauf der jeweiligen Lösungen wurde durch entsprechende Verbindungsbohrungen in den Dichtrahmen und den Endplatten sowie durch den Anschluß an die entsprechenden Pumpkreise erreicht.

Bei den Membranen vom Typ Nafion handelt es sich um Copolymere aus Tetrafluorethylen und einer perflurierten ungesättigten aliphatischen Sulfonsäure, z.B. Perfluora-3,6-dioxa-4-methyloct-7-en-sulfonsäure, und die Membranen vom Typ des Selemions bestehen aus vernetztem Polystyrol, welches Sulfonsäuregruppen bzw. quartäre Ammoniumgruppen als ionenaustauschende Gruppen tragen.

### b) Lösungen

Die Kreisläufe der Elektrodialyseapparatur wurden mit den folgenden Lösungen beschickt:
- Diluat: Es wurde eine wäßrige Lösung vom ungefähren pH-Wert 9,5 verwendet, die 1,4-Bis-aminooxy-butan und Natriumchlorid enthielt und die durch Neutralisation der wäßrigen Lösung des Hydrochlorides von 1,4-Bis-aminooxy-butan mit Natronlauge erhalten worden war. Die hierbei ausgefallenen Nebenprodukte der Spaltung des Butylen-1,4-bis-acetonoximethers wurden mit Aktivkohle vor der Elektrodialyse abfiltriert.
- Konzentrat: 0,9-1,8 kg vollentsalztes Wasser mit 0,5 Gew.-% Natriumchlorid.
- Elektrodenspüllösung: 2 kg einer Na₂SO₄-Lösung mit einer Leitfähigkeit von ca. 100 mS/cm

### c) Durchführung

Die Lösungen wurden im Kreis über die ED-Zelle gepumpt und bei ca. 35°C mit einer Zellspannung bis zu 30 V sowie mit einem Elektrodialysestrom bis zu 3 A elektrodialysiert.

Es wurden zwei Versuche vorgenommen, und zwar mit 1 bzw. 2 kg Diluat. Diluat 1 enthielt 9,6 Gew.-% 1,4-Bis-aminooxy-butan und 7,4 Gew.-% Natriumchlorid, bei Diluat 2 waren es 9,3 Gew.-% 1,4-Bis-aminooxy-butan und 6 Gew.-% Natriumchlorid. Die Versuche wurden nach 140 bzw. 260 Minuten abgebrochen, nachdem die Salzmenge des Diluats um 95 bzw. 97 % abgereichert war. Die Verluste an 1,4-Bis-aminooxy-butan betrugen in beiden Fällen nur rund 5 %.

### Beispiel 4

### Herstellung einer Polymerdispersion A

Die Dispersion wurde bei 85°C durch Zugabe von Zulauf 1 im Laufe von 180 und Zulauf 2 im Laufe von 210 Minuten zu einer vorab 15 Minuten auf 80°C erhitzten Vorlage und Nachreaktion von 60 Minuten bei 80°C hergestellt.

### Zulauf 1, Emulsion aus

340 g entsalztem Wasser
650 g Vinylpropionat
270 g tert.-Butylacrylat
80 g n-Butylacrylat
10 g Acrylsäure
20 g einer 50 gew.-%igen wäßrigen Lösung von Acrylamid
80 g einer 50 gew.-%igen wäßrigen Lösung von Diacetonacrylamid (N-(1,1-Dimethyl-3-oxo-butyl)-acrylamid)
und einem Emulgatorgemisch aus
100 g einer 20 gew.-%igen wäßrigen Lösung des Natriumsalzes eines mit 2,5 Ethylenoxideinheiten veretherten ₁₂-Fettalkoholsulfats und
75 g einer 20 gew.-%igen wäßrigen Lösung eines mit 18 Ethylenoxideinheiten veretherten C₁₆-/C₁₈-Fettalkoholgemisches

### Zulauf 2

100 g entsalztes Wasser
5 g Natriumpersulfat

### Vorlage

365 g entsalztes Wasser
80 g Zulauf 1
10,5 g Zulauf 2
2,5 g Natriumacetat (Puffer)
2 g Acrylsäure

### Beispiel 5

### Herstellung einer Polymerdispersion B

Die Dispersion wurde bei 85°C durch Zugabe von Zulauf 1 im Laufe von 120 und Zulauf 2 im Laufe von 150 Minuten zu einer vorab 15 Minuten auf 85°C erhitzten Vorlage und Nachreaktion von 60 Minuten bei 85°C hergestellt.

### Zulauf 1, Emulsion aus

108 g entsalztem Wasser
400 g Ethylacrylat
90 g Methylmethacrylat und
50 g einer 20 gew.-%igen wäßrigen Lösung von Diacetonacrylamid (N-(1,1-Dimethyl-3-oxo-butyl)-acrylamid)
und einem Emulgatorgemisch aus
50 g einer 20 gew.-%igen wäßrigen Lösung von p-Dodecyldiphenylether-disulfonsaure-dinatriumsalz und
50 g einer 20 gew.-%igen wäßrigen Lösung eines mit 50 Ethylenoxideinheiten verethertem p-Isononylphenol

### Zulauf 2

100 g entsalztes Wasser
3 g Natriumpersulfat

### Vorlage

200 g entsalztes Wasser
37 g Zulauf 1
20 g Zulauf 2

### Beispiel 6

### Herstellung vernetzbarer Dispersionen und Filmen hiervon

Die Dispersion A gemäß den Beispiel 4 wurde auf einen Feststoffgehalt von 50 Gew.-% gestellt. 100 g dieser Dispersion wurde mit 9,9 g einer wäßrigen Lösung versetzt, die 0,95 g 1,4-Bis-aminooxy-butan (= 0,7 mol/mol Carbonylgruppen in Polymerisat) enthielt und die gemäß Beispiel 3 bis auf einen Restgehalt von rd. 8 mol-% Natriumchlorid, bezogen auf 1,4-Bis-aminooxy-butan, entsalzt worden war.

Die so erhaltene gebrauchsfertige Dispersion blieb über einen Beobachtungszeitraum von 4 Tagen stabil, und sie lieferten nach Auftragung auf eine Glasplatte bei Raumtemperatur einen klaren vernetzten Film.

Zum Vergleich wurde diese Dispersion mit der gleichen Menge der wäßrigen Lösung des 1,4-Bis-aminooxy-butans versetzt, die jedoch noch nicht entsalzt worden war.

Diese Dispersion war nach etwa einem Tag koaguliert.

Analog verhielt sich die Dispersion B, bei der das Molverhältnis des Vernetzers zur Anzahl der Carbonylgruppen im Polymerisat 0,5:1 betrug.

## Patentansprüche

1. Verfahren zur Herstellung von weitgehend salzfreien wäßrigen Lösungen von Bis-aminooxy-alkanen (I), dadurch gekennzeichnet, daß man
a) ein Dihalogenalkan (II) mit einem Ketoxim (III) in der wäßrigen Lösung einer starken Mineralbase in den Alkylenbis-oximether (IV) überführt,
b) den Alkylen-bis-oximether (IV) mit einer starken Mineralsäure und Wasser in das Keton und das mineralsaure Salz des Bis-aminooxy-alkans (I) spaltet und das Keton in an sich bekannter Weise abtrennt,
c) die verbleibende wäßrige Lösung zur Freisetzung des Bis-aminooxy-alkans (I) mit einem Alkalimetallhydroxid versetzt und
d) aus der so erhaltenen wäßrigen Lösung die ionischen Bestandteile durch Elektrodialyse entfernt.

2. Verfahren nach Anspruch 1, dadurch gekennzeichnet, daß man als Dihalogenalkan (II) ein unverzweigtes α,ω-Dichlor- oder α,ω-Dibrom-C₂- bis C₁₂-alkan einsetzt.

3. Verfahren nach Anspruch 1 oder 2, dadurch gekennzeichnet, daß man als Ketoxim (III) in Stufe (a) Acetonoxim verwendet.

4. Verfahren nach den Ansprüchen 1 bis 3, dadurch gekennzeichnet, daß man als starke Mineralbase in Stufe (a) Natriumhydroxid oder Calciumhydroxid verwendet.

5. Verfahren nach den Ansprüchen 1 bis 4, dadurch gekennzeichnet, daß man als starke Mineralsäure in Stufe (b) Salzsäure verwendet.

6. Verfahren zur Herstellung von Alkylen-bis-acetonoximethern durch Umsetzung eines Dihalogenalkans (II) mit Acetonoxim in Gegenwart einer starken wäßrigen Mineralbase, dadurch gekennzeichnet, daß man die Umsetzung in Gegenwart eines Phasentransferkatalysators sowie ohne Mitverwendung nennenswerter Mengen eines Lösungsmittels vornimmt.

7. Verfahren zur Herstellung von Hydrochloriden von Bis-aminooxy-alkanen (I), dadurch gekennzeichnet, daß man einen Alkylen-bis-acetonoximether mit wäßriger Salzsäure hydrolysiert und das entstehende Aceton laufend aus dem Reaktionsgemisch entfernt.

8. Verfahren zur Herstellung von weitgehend salzfreien wäßrigen Lösungen von Bis-aminooxy-alkanen (I), dadurch gekennzeichnet, daß man Alkalimetallsalze enthaltende wäßrige Lösungen eines Bis-aminooxy-alkans (I) der Elektrodialyse unterwirft.

9. Verfahren nach Anspruch 8, dadurch gekennzeichnet, daß man die Elektrodialyse bei einem pH-Wert durchführt, der gleich oder größer ist als der pH-Wert der wäßrigen Lösung des freien Bis-aminooxy-alkans (I).

10. Verwendung der weitgehend salzfreien wäßrigen Lösungen von Bis-aminooxy-alkanen (I) gemäß den Ansprüchen 1 bis 5, 8 oder 9 als Vernetzungsmittel enthaltendem Zusatz zu Dispersionen von vernetzbaren Polymeren, welche Carbonylgruppen in Aldehyd- oder Ketonfunktion als funktionelle Gruppen tragen.

11. Wäßrige Dispersionen, enthaltend
a) ein vernetzbares Polymeres mit Carbonylgruppen in Aldehyd- oder Ketonfunktion als funktionellen Gruppen und
b) ein Bis-aminooxy-alkan (I) in Form der freien Base,
wobei der Anteil von mineralsauren Salzen von Alkalimetallen in der Dispersion (I) unter 20 mol-%, bezogen auf die Menge des Bis-aminooxy-alkans (I), liegt.
